# EUROPEAN PATENT SPECIFICATION

(11) **EP 1 255 535 B1**
(45) Date of publication and mention of the grant of the patent: **26.04.2006**
(21) Application number: 00987947.9
(22) Date of filing: 20.12.2000
(51) Int. Cl.: A61K 9/20, A61K 31/315

(54) **CONTROLLED RELEASE PHARMACEUTICAL COMPOSITION CONTAINING TRAMADOL HYDROCHLORIDE**
ARZNEIZUBEREITUNG MIT KONTROLLIERTER FREISETZUNG ENTHALTEND TRAMADOL HYDROCHLORID
COMPOSITION PHARMACEUTIQUE A LIBERATION REGULEE CONTENANT DU CHLORHYDRATE DE TRAMADOL ET SON PROCEDE DE PREPARATION

(30) Priority: 28.12.1999 SK 186899
(43) Date of publication of application: 13.11.2002
(73) Proprietor: Zentiva, a.s., 920 27 Hlohovec (SK)
(72) Inventor: RAZUS, L'uboslav, 920 01 Hlohovec (SK); SEDLAROV , Helena, 920 01 Hlohovec (SK); VARGA, Ivan, 920 01 Hlohovec (SK); GATTNAR, Ondrej, 831 01 Bratislava (SK); ZEMANEK, Marián, 811 03 Bratislava (SK)
(74) Representative: Flaccus, Rolf-Dieter
(86) International application number: PCT/SK2000/000027
(87) International publication number: WO 2001/047497

(56) References cited:
- EP-A- 1 020 183
- WO-A-98/14176
- US-A- 5 601 842
- DATABASE CHEMABS [Online] CHEMICAL ABSTRACTS SERVICE, COLUMBUS, OHIO, US; OBAIDAT, AIMAN A.: "Evaluation of the mechanism of release of a water soluble drug from a waxy inert matrix" retrieved from STN Database accession no. 133:125074 XP000990961 & ACTA PHARM. TURC. ( 1999 ), 41(4), 199-202 ,

## Description

### Technical Field

The invention belongs to the pharmaceutical field, it relates to the composition and the method of preparing of an oral therapeutic preparation in the form of controlled release tablets containing the active ingredient tramadol hydrochloride.

### Background art

Tramadol hydrochloride containing therapeutic compositions in the form of controlled release tablets are described in SK patent No. 280496, according to which a formulation is prepared by melting a mixture of the drug and a hydrophobic or hydrophilic carrier in high performance homogenization devices with heating and cooling facilities. The molten, homogenized mixture is cooled down; particle size of the agglomerates is adjusted while undercooling the mixture in order to obtain desirable physical parameters for mechanic dividing and adjustment of the particle size for further processing.

As described in SK patent No. 280496, the meltable carriers include, e.g., waxes, hydrogenated vegetable oils and higher fatty acid esters with glycerol.

Document EP 0 624 366 A1 describes a formulation containing 50-800 mg tramadol, i.e. controlled release tablets, which are produced in a similar manner, or in the form of film coated spheres (agglomerates).

Drawbacks of the above preparation of solid formulations containing tramadol hydrochloride based on meltable hydrophobic or hydrophilic carrier include, in addition to energy and time consumption, necessity of special equipment. Another drawback is the surface finish of the tablets, which requires further special equipment and technology, and dividing of the tablets for dosage is not possible.

### Disclosure of the Invention

This invention overcomes said drawbacks by a new, simpler, and low time and energy consuming method of preparing tablets containing 100 to 200 mg tramadol hydrochloride, not requiring special production equipment. Manufacture in the below described manner is practicable in pharmaceutical plants without need of one-purpose equipment.

The tablets made by the method of the invention fulfil the requirements for the release pattern and do not require any further finish by, e.g. film coating, which would influence the release rate.

The principle of the manufacture is a balanced mixture of the drug and the adjuvants, prepared by simple granulation, drying of the granulate, admixing further adjuvants facilitating the tabletting process without need of further treatment of the compressed articles. Thus obtained tablets are physically and chemically stable, easily adjustable and ensure necessary optimal course of release of the active substance into the organism over the required time period even after possible dividing of the tablets.

The invention is disclosed in independent claims 1 and 12.

The controlled release formulation according to this invention contains other adjuvants in addition to the active substance:
a) Micronized esters of glycerol with higher fatty acids, preferably docosanoic acid glyceryl ester. They are of a particle size from 1 to 100 micrometers, preferably of a distribution the size of which is 1.5-60 micrometers in the range of 90 %. It has been determined experimentally in laboratory development that the most preferred content of the higher fatty acid glyceryl esters for the targeted drug release and optimal physical characteristics is from 10 to 53 % by weight, preferably, from 28 to 47 % by weight.
b) Pharmaceutically applicable alkali salts of phosphoric acid, preferably calcium phosphate dihydrate, in amounts of from 20 to 41 % by weight, preferably from 24 to 39 % by weight.
c) Non-ionic polymers of vinylpyrrolidone of relative molecular weight from 9000 to 90000, preferably 25000 to 30000, in amounts from 1.15 to 1.75 % by weight, preferably from 1.3 to 1.55 % by weight.
d) Substances facilitating tabletting process from the group of salts of higher fatty acids with alkaline earth metals, preferably magnesium stearate, in amounts of 1.5 to 3.2 % by weight, preferably from 1.8 to 2.8 % by weight, and silicon dioxide, preferably colloidal silica, in amounts of from 1 to 3 % by weight, preferably from 1.1 to 2.1 % by weight.

The manufacturing process of this invention consists in mixing the active substance in admixture with micronized higher fatty acid glycerol ester, preferably with glyceryl ester of docosanoic acid having a particle size of from 1 - 100 micrometers, preferably 1.5 - 60 micrometers, in an amount of from 10 % to 53 % by weight, preferably from 28 % to 47 % by weight, along with alkali salts of phosphoric acid, preferably calcium phosphate dihydrate, in an amount of from 20 % to 41 % by weight, preferably from 24 % to 39 % by weight. This mixture is moistened with a solution of a non-ionic vinylpyrrolidone polymer having a relative molecular weight of from 9,000 to 90,000, preferably from 25,000 to 30,000, in an amount of 1.15 to 1.75 % by weight, preferably from 1.3 to 1.55 % by weight, in a mixture of water and ethyl alcohol in an amount of from 30 % to 70 % by weight, preferably from 40 % to 60 % by weight.

The mixture is agitated while agglomerating. The obtained agglomerate is dried in a suitable manner either by fluidisation, in a chamber or in vacuo such that the mixture contain from 0.2-1.5 %, preferably from 0.5 to 1.2 % moisture.

The dried agglomerate is adjusted to a particle size that complies with the tabletting process, and materials from the group of salts of higher fatty acids with alkaline earth metals, preferably magnesium stearate, in an amount of from 1.5 % to 3.2 % by weight, preferably from 1.8 % to 2.8 % by weight, and of silicon oxides, preferably colloidal silica, in an amount of from 1 % to 1.3 % by weight, preferably from 1.1 % to 2.1 % by weight, are added.

The mixture is agitated until homogeneous.

The mixture is tabletted, the break resistance of tablets ranging from 40 to 110 N, preferably from 50 to 90 N, for tablets of round, lenticular, oblong or other shapes.

Thus prepared tablets can be adjusted into commonly useful types of packages such as glass, plastics, metal packages and combinations thereof.

### Examples

The following examples are intended to illustrate the invention without limiting its scope.

### Example 1

### a) pharmaceutical composition, containing in 1 tablet:

| | | |
|---|---|---|
| Tramadol hydrochloride | 0.1000 g | 21.74 % |
| Glyceryl ester of docosanoic acid | 0.1700 g | 36.96 % |
| Calcium phosphate dihydrate | 0.1770 g | 38.48 % |
| Polyvinylpyrrolidone | 0.0070 g | 1.52 % |
| Colloidal silica | 0.0060 g | 1.30 % |

### b) process for its preparation:

The active ingredient in admixture with micronized ester of glycerol with docosanoic acid having the particle size of from 1.5 - 60 micrometers in the amount of 36.96 % by weight is agitated in a suitable type of pharmaceutical granulator such as Diosna, along with calcium phosphate dihydrate in the amount of 38.48 % by weight, for 3 minutes.

Then the mixture is, under constant agitation, moistened with a solution of non-ionic vinylpyrrolidone polymer having the relative molecular weight of 25,000 in the amount of 1.52 % by weight in 60% ethanol.

The mixture is agitated, agglomerate thus being formed.

The prepared agglomerate is discharged from the granulator into the vessel of a fluidising drying device such as Glatt or Aeromatic and is dried at the temperature of fed air 55 °C until the temperature of the effluent air reaches 42 °C. At that point the product reaches residual moisture from 0.5 % to 1.2 %.

The particle size of the dried agglomerate is adjusted by passing through a screen having the mesh side of 1.25 mm on an oscillating device such as Frewitt. The adjusted agglomerate is transferred into a suitable type of pharmaceutical homogenizer of the shape of cube or bulb, colloidal silica in the amount of 1.3% by weight is added and agitated until homogeneous. The obtained mixture is tabletted in rotary tabletting machines of the type such as Manesty, Kilian, Fette etc. into round-shaped, biconvex tablets.

**Tablet parameters:**

| **Appearance** | White to off-white, smooth, biconvex, round with dividing groove |
|---|---|
| **Tablet diameter (mm)** | 11 |
| **Tablet height (mm)** | 4.75 |
| **Tablet average weight (g)** | 0.460 |
| **Tablet break resistance (N)** | 50-70 |

Dissolution of the active ingredient according to this invention as a function of time and its comparison with the product according to EP 0 624 366 A1:

**Apparatus Pharmatest type PTWS 3:**

| **Dissolution medium Water** (Temperature 37 °, volume 600 ml, 100 rpm) | **Time** | | | |
|---|---|---|---|---|
| | **Amount of released active ingredient, %** | | | |
| | after 1 hour | after 3 hours | after 5 hours | after 8 hours |
| Tramadol 100-SL | 38.90 | 63.46 | 77.09 | 90.44 |
| Tramal ® Retard 100 | 35.73 | 65.80 | 81.75 | 93.75 |

### Example 2

### a) pharmaceutical composition, containing in 1 tablet:

| | | |
|---|---|---|
| Tramadol hydrochloride | **0.1500** g | 21.74% |
| Glyceryl ester of docosanoic acid | 0.2550 g | 36.96 % |
| Calcium phosphate dihydrate | 0.2655 g | 38.48 % |
| Polyvinylpyrrolidone | 0.0105 g | 1.52 % |
| Colloidal silica | 0.0090 g | 1.30 % |

### b) process for its preparation:

The active ingredient in admixture with micronized ester of glycerol with docosanoic acid having the particle size of from 1.5 - 60 micrometers in the amount of 36.96 % by weight is agitated in a suitable type of pharmaceutical granulator such as Diosna, along with calcium phosphate dihydrate in the amount of 38.48 % by weight, for 3 minutes.

Then the mixture is, under constant agitation, moistened with a solution of non-ionic vinylpyrrolidone polymer having the relative molecular weight of 25,000 in the amount of 1.52 % by weight in 60% ethanol.

The mixture is agitated, agglomerate thus being formed.

The prepared agglomerate is discharged from the granulator into the vessel of a fluidising drying device such as Glatt or Aeromatic and is dried at the temperature of fed air 55 °C until the temperature of the effluent air reaches 42 °C. At that point the product reaches residual moisture from 0.5 % to 1.2 %.

The particle size of the dried agglomerate is adjusted by passing through a screen having the mesh side of 1.30 mm on an oscillating device such as Frewitt. The adjusted agglomerate is transferred into a suitable type of pharmaceutical homogenizer of the shape of cube or bulb, colloidal silica in the amount of 1.30% by weight is added and agitated until homogeneous. The obtained mixture is tabletted in rotary tabletting machines of the type such as Manesty, Kilian, Fette etc. into round-shaped, flat tablets with dividing groove.

**Tablet parameters:**

| **Appearance** | Off-white, smooth, flat with dividing groove |
|---|---|
| **Tablet diameter (mm)** | 12 |
| **Tablet height (mm)** | 4.2-4.5 |
| **Tablet average weight (g)** | 0.690 |
| **Tablet break resistance (N)** | 58-80 |

Dissolution of the active ingredient according to this invention as a function of time and its comparison with the product according to EP 0 624 366 A1:

**Apparatus Pharmatest type PTWS 3:**

| **Dissolution medium** Water (Temperature 37 °, volume 600 ml, 100 rpm) | **Time** | | | |
|---|---|---|---|---|
| | **Amount of released active ingredient, %** | | | |
| | after 1 hour | after 3 hours | after 5 hours | after 8 hours |
| Tramadol 150-SL | 40.32 | 67.12 | 80.24 | 95.09 |
| Tramal ® Retard 150 | 34.74 | 65.2 | 83.36 | 95.58 |

### Example 3

### a) pharmaceutical composition, containing in 1 tablet:

| | | |
|---|---|---|
| Tramadol hydrochloride | **0.1500** g | 29.41 % |
| Glyceryl ester of docosanoic acid | 0.1700 g | 33.33 % |
| Calcium phosphate dihydrate | 0.1770 g | 34.71 % |
| Polyvinylpyrrolidone | 0.0070 g | 1.37 % |
| Colloidal silica | 0.0060 g | 1.18 % |

### b) process for its preparation:

The active ingredient in admixture with micronized ester of glycerol with docosanoic acid having the particle size of from 1.5 - 60 micrometers in the amount of 33.33 % by weight is agitated in a suitable type of pharmaceutical granulator such as Diosna, along with calcium phosphate dihydrate in the amount of 34.71 % by weight, for 3 minutes.

Then the mixture is, under constant agitation, moistened with a solution of non-ionic vinylpyrrolidone polymer having the relative molecular weight of 25,000 in the amount of 1.37 % by weight in 60% ethanol.

The mixture is agitated, agglomerate thus being formed.

The prepared agglomerate is discharged from the granulator into the vessel of a fluidising drying device such as Glatt or Aeromatic and is dried at the temperature of fed air 55 °C until the temperature of the effluent air reaches 42 °C. At that point the product reaches residual moisture from 0.5 % to 1.2 %.

The particle size of the dried agglomerate is adjusted by passing through a screen having the mesh side of 1.25 mm on an oscillating device such as Frewitt. The adjusted agglomerate is transferred into a suitable type of pharmaceutical homogenizer of the shape of cube or bulb, colloidal silica in the amount of 1.18% by weight is added and agitated until homogeneous.

The obtained mixture is tabletted in rotary tabletting machines of the type such as Manesty, Kilian, Fette etc. into round-shaped, flat tablets with dividing groove.

**Tablet parameters:**

| **Appearance** | Off-white, smooth, flat with dividing groove |
|---|---|
| **Tablet diameter (mm)** | 11 |
| **Tablet height (mm)** | 4.41 |
| **Tablet average weight (g)** | 0.510 |
| **Tablet break resistance (N)** | 58-75 |

Dissolution of the active ingredient according to this invention as a function of time and its comparison with the product according to EP 0 624 366 A1.

**Apparatus Pharmatest type PTWS 3:**

| **Dissolution medium** Water (Temperature 37 °, volume 600 ml, 100 rpm) | **Time** | | | |
|---|---|---|---|---|
| | **Amount of released active ingredient, %** | | | |
| | after 1 hour | after 3 hours | after 5 hours | after 8 hours |
| Tramadol 150-SL | 42.30 | 68.7 | 82.90 | 95.15 |
| Tramal ® Retard 150 | 34.74 | 65.2 | 83.36 | 95.58 |

### Example 4

### a) pharmaceutical composition, containing in 1 tablet:

| | | |
|---|---|---|
| Tramadol hydrochloride | **0.2000** g | 26.85 % |
| Glyceryl ester of docosanoic acid | 0.3400 g | 45.64 % |
| Calcium phosphate dihydrate | 0.1800 g | 24.16 % |
| Polyvinylpyrrolidone | 0.0100 g | 1.34 % |
| Magnesium stearate | 0.0150 g | 2.010 % |

### b) process for its preparation:

The active ingredient in admixture with micronized ester of glycerol with docosanoic acid having the particle size of from 1.5 - 60 micrometers in the amount of 45.64 % by weight is agitated in a suitable type of pharmaceutical granulator such as Diosna, along with calcium phosphate dihydrate in the amount of 24.16 % by weight, for 3 minutes.

Then the mixture is, under constant agitation, moistened with a solution of non-ionic vinylpyrrolidone polymer having the relative molecular weight of 25,000 in the amount of 1.34 % by weight in 60% ethanol.

The mixture is agitated, agglomerate thus being formed.

The prepared agglomerate is discharged from the granulator into the vessel of a fluidising drying device such as Glatt or Aeromatic and is dried at the temperature of fed air 55 °C until the temperature of the effluent air reaches 42 °C. At that point the product reaches residual moisture from 0.5 % to 1.2 %.

The particle size of the dried agglomerate is adjusted by passing through a screen having the mesh side of 1.25 mm on an oscillating device such as Frewitt. The adjusted agglomerate is transferred into a suitable type of pharmaceutical homogenizer of the shape of cube or bulb, magnesium stearate in the amount of 2.01% by weight is added and agitated until homogeneous.

The obtained mixture is tabletted in rotary tabletting machines of the type such as

Manesty, Kilian, Fette etc. into smooth, flat tablets with dividing groove.

**Tablet parameters:**

| **Appearance** | White to off-white, smooth, flat with dividing groove |
|---|---|
| **Tablet diameter (mm)** | 13 |
| **Tablet height (mm)** | 4.88 |
| **Tablet average weight (g)** | 0.745 |
| **Tablet break resistance (N)** | 70-90 |

Dissolution of the active ingredient according to this invention as a function of time and its comparison with the product according to EP 0 624 366 A1:

**Apparatus Pharmatest type PTWS 3:**

| **Dissolution medium** Water (Temperature 37 °, volume 600 ml, 100 rpm) | **Time** | | | |
|---|---|---|---|---|
| | **Amount of released active ingredient, %** | | | |
| | after 1 hour | after 3 hours | after 5 hours | after 8 hours |
| Tramadol 200-SL | 33.48 | 56.53 | 69.82 | 82.7 |
| Tramal ® Retard 200 | 35.60 | 66.50 | 83.90 | 95.8 |

### Example 5

### a) pharmaceutical composition, containing in 1 tablet:

| | | |
|---|---|---|
| Tramadol hydrochloride | **0.2000** g | 21.74 % |
| Glyceryl ester of docosanoic acid | 0.3400 g | 36.96 % |
| Calcium phosphate dihydrate | 0.3540 g | 38.48 % |
| Polyvinylpyrrolidone | 0.0140 g | 1.52 % |
| Colloidal silica | 0.0120 g | 1.30 % |

### b) process for its preparation:

The active ingredient in admixture with micronized ester of glycerol with docosanoic acid having the particle size of from 1.5 - 60 micrometers in the amount of 36.9 % by weight is agitated in a suitable type of pharmaceutical granulator such as Diosna, along with calcium phosphate dihydrate in the amount of 38.48 % by weight, for 3 minutes.

Then the mixture is, under constant agitation, moistened with a solution of non-ionic vinylpyrrolidone polymer having the relative molecular weight of 25,000 in the amount of 1.52 % by weight in 60% ethanol.

The mixture is agitated, agglomerate thus being formed.

The prepared agglomerate is discharged from the granulator into the vessel of a fluidising drying device such as Glatt or Aeromatic and is dried at the temperature of fed air 55 °C until the temperature of the effluent air reaches 42 °C. At that point the product reaches residual moisture from 0.5 % to 1.2 %.

The particle size of the dried agglomerate is adjusted by passing through a screen having the mesh side of 1.25 mm on an oscillating device such as Frewitt. The adjusted agglomerate is transferred into a suitable type of pharmaceutical homogenizer of the shape of cube or bulb, colloidal silica in the amount of 1.30% by weight is added and agitated until homogeneous.

The obtained mixture is tabletted in rotary tabletting machines of the type such as Manesty, Kilian, Fette etc. into flat, oblong tablets with dividing groove.

**Tablet parameters:**

| **Appearance** | Off-white, smooth, oblong with dividing groove |
|---|---|
| **Tablet diameter (mm)** | 18 |
| **Tablet height (mm)** | 6.6 |
| **Tablet average weight (g)** | 0.920 |
| **Tablet break resistance (N)** | 70-90 |

Dissolution of the active ingredient according to this invention as a function of time and its comparison with the product according to EP 0 624 366 A1:

**Apparatus Pharmatest type PTWS 3:**

| **Dissolution medium** Water (Temperature 37 °, volume 600 ml, 100 rpm) | **Time** | | | |
|---|---|---|---|---|
| | **Amount of released active ingredient, %** | | | |
| | after 1 hour | after 3 hours | after 5 hours | after 8 hours |
| Tramadol 200-SL | 30.70 | 53.40 | 66.50 | 79.60 |
| Tramal ® Retard 200 | 35.60 | 66.50 | 83.90 | 95.8 |

### Industrial Applicability

The invention can be employed in the pharmaceutical industry in obtaining controlled release therapeutic preparations, containing tramadol hydrochloride. Said preparations are indicated in therapy of acute and chronic moderate to strong pain of various origins, in particular in surgery, obstetrics, oncology, rheumatology, orthopaedics, after stomatological operations, in neurology and other fields. It is useful also for pain in ischaemic diseases (such as in myocardial infarction and in leg ischaemias).

## Claims

1. A controlled release pharmaceutical composition containing tramadol hydrochloride **characterized in that** it contains 100 to 200 mg of the active ingredient in admixture with micronized glyceryl ester of docosanoic acid having the particle size from 1 to 100 micrometers in an amount of from 10 to 53 % by weight, with alkali salts of phosphoric acid, non-ionic vinylpyrrolidone polymers, and with substances from the group including salts of higher fatty acids with alkaline earth metals and silicon oxides.

2. The controlled release pharmaceutical composition containing tramadol hydrochloride according to claim 1 **characterized in that** 90 % of the particle size of the docosanoic acid glyceryl ester ranges from 1.5 to 60 micrometers.

3. The controlled release pharmaceutical composition containing tramadol hydrochloride according to claims 1 and 2 **characterized in that** the contents of the docosanoic acid glyceryl ester is in the range of from 28 to 47 % by weight.

4. The controlled release pharmaceutical composition containing tramadol hydrochloride according to claim 1 **characterized in that** it contains alkali salts of phosphoric acid, preferably calcium phosphate dihydrate in an amount from 20 to 41 % by weight.

5. The controlled release pharmaceutical composition containing tramadol hydrochloride according to claim 4 **characterized in that** it contains calcium phosphate dihydrate preferably in an amount from 24 to 39 % by weight.

6. The controlled release pharmaceutical composition containing tramadol hydrochloride according to claims 1 and 4 **characterized in that** it contains non-ionic vinylpyrrolidone polymers having a relative molecular weight of 9000 to 90,000 in an amount of 1.15 to 1.75 % by weight.

7. The controlled release pharmaceutical composition containing tramadol hydrochloride according to claim 6 **characterized in that** it contains non-ionic vinylpyrrolidone polymers having a relative molecular weight of preferably 25,000 to 30,000 in amount of preferably from 1.3 to 1.55 % by weight.

8. The controlled release pharmaceutical composition containing tramadol hydrochloride according to claims 1, 4 and 6 **characterized in that** it contains substances from the group salts of higher fatty acids with alkaline earth metals in an amount of 1.5 to 3.2 % by weight.

9. The controlled release pharmaceutical composition containing tramadol hydrochloride according to claim 8 **characterized in that** it contains substances from the group salts of higher fatty acids with alkaline earth metals, preferably magnesium stearate in an amount of preferably from 1.8 to 2.8 % by weight.

10. The controlled release pharmaceutical composition containing tramadol hydrochloride according to claims 1, 4, 6 and 8 **characterized in that** it contains silicon oxides in an amount of 1 to 3.0 % by weight.

11. The controlled release pharmaceutical composition containing tramadol hydrochloride according to claim 10 **characterized in that** it contains silicon oxides, preferably colloidal silica in an amount of 1.1 to 2.1 % by weight.

12. A method of preparing the pharmaceutical composition according to claims 1 to 11 **characterized in that** the active ingredient is agitated in admixture with the glyceryl ester of docosanoic acid of a particle size from 1 to 100 µm in an amount of 10 to 53 % by weight and with alkali salts of phosphoric acid wherein said admixture is moisturized with a solution of a non-ionic vinylpyrrolidone polymer in a mixture of water and ethyl alcohol and the mixture is agglomerated.

13. The method of preparing the pharmaceutical composition according to claim 12 **characterized in that** the active ingredient is agitated in admixture with the glyceryl ester of docosanoic acid of a particle size from 1.5 to 60 µm, in an amount of 28 to 47 % by weight, along with alkali salts of phosphoric acid, preferably calcium phosphate dihydrate, in an amount of 20 to 41 % by weight, preferably of 24 to 39 % by weight.

14. The method of preparing the pharmaceutical composition according to claims 12 and 13 **characterized in that** the mixture of the active ingredient, said glyceryl ester of docosanoic acid and alkali salts of phosphoric acid is moisturized with a solution of a non-ionic vinylpyrrolidone polymer of a relative molecular weight from 9000 to 90,000, preferably 25,000 to 30,000, in an amount of 1.15 to 1.75 % by weight, preferably 1.3 to 1.55 % by weight.

15. The method of preparing the pharmaceutical composition according to claims 12 to 14 **characterized in that** the mixture of the active ingredient, said glyceryl ester of docosanoic acid and alkali salts of phosphoric acid is moisturized with a solution of a non-ionic vinylpyrrolidone polymer in a mixture of water and ethyl alcohol in an amount of 30 to 70 % by weight, preferably 40 to 60 % by weight.

16. The method of preparing the pharmaceutical composition according to claims 12 to 15 **characterized in that** the mixture is agitated and agglomerated, the obtained agglomerate being dried in a suitable manner by fluidising drying, chamber drying or vacuum drying such that the mixture contains from 0.2 to 1.5, preferably from 0.5 to 1.2 % moisture.

17. The method of preparing the pharmaceutical composition according to claims 12 to 16 **characterized in that** the dried agglomerate is adjusted to a particle size convenient for tabletting process and substances from the group of salts of higher fatty acids with alkaline earth metals and/or silicon oxides are admixed, the mixture being agitated until homogeneous.

18. The method of preparing the pharmaceutical composition according to claim 17 **characterized in that** the dried agglomerate is adjusted to a particle size convenient for tabletting process and admixed are substances from the group of salts of higher fatty acids with alkaline earth metals, preferably magnesium stearate in an amount of 1.5 to 3.2 % by weight, preferably 1.8 to 2.8 % by weight, and/or silicon oxides, preferably colloidal silica in an amount from 1 to 3 % by weight, preferably from 1.1 to 2.1 % by weight.

19. The method of preparing the pharmaceutical composition according to claims 12 to 18 **characterized in that** the prepared mixture is tabletted to tablets having a lenticular, flat oblong or other shapes such that break resistance of the tablets ranges between 40 and 110 N, preferably from 50 to 90 N.

## Patentansprüche

1. Arzneizusammensetzung mit kontrollierter Freisetzung enthaltend Tramadolhydrochlorid, **dadurch gekennzeichnet, dass** sie 100 bis 200 mg des Wirkstoffs in Mischung mit mikronisiertem Glycerinester der Docosansäure mit einer Partikelgröße von 1 bis 100 Mikrometer in einer Menge von 10 bis 53 Gew.-%, mit Alkalisalzen der Phosphorsäure, nichtionischen Vinylpyrrolidonpolymeren und mit Substanzen aus der Gruppe enthaltend Salze höherer Fettsäuren mit Erdalkalimetallen und Siliziumoxide enthält.

2. Arzneizusammensetzung mit kontrollierter Freisetzung enthaltend Tramadolhydrochlorid nach Anspruch 1, **dadurch gekennzeichnet, dass** 90 % der Partikelgröße des Docosansäureglycerinesters im Bereich von 1,5 bis 60 Mikrometer liegt.

3. Arzneizusammensetzung mit kontrollierter Freisetzung enthaltend Tramadolhydrochlorid nach den Ansprüchen 1 und 2, **dadurch gekennzeichnet, dass** der Gehalt an Docosansäureglycerinester im Bereich von 28 bis 47 Gew.-% liegt.

4. Arzneizusammensetzung mit kontrollierter Freisetzung enthaltend Tramadolhydrochlorid nach Anspruch 1, **dadurch gekennzeichnet, dass** sie Alkalisalze der Phosphorsäure, vorzugsweise Calciumphosphatdihydrat, in einer Menge von 20 bis 41 Gew.-% enthält.

5. Arzneizusammensetzung mit kontrollierter Freisetzung enthaltend Tramadolhydrochlorid nach Anspruch 4, **dadurch gekennzeichnet, dass** sie Calciumphosphatdihydrat vorzugsweise in einer Menge von 24 bis 39 Gew.-% enthält.

6. Arzneizusammensetzung mit kontrollierter Freisetzung enthaltend Tramadolhydrochlorid nach den Ansprüchen 1 und 4, **dadurch gekennzeichnet, dass** sie nichtionische Vinylpyrrolidonpolymere mit einem relativen Molekulargewicht von 9000 bis 90.000 in einer Menge von 1,15 bis 1,75 Gew.-% enthält.

7. Arzneizusammensetzung mit kontrollierter Freisetzung enthaltend Tramadolhydrochlorid nach Anspruch 6, **dadurch gekennzeichnet, dass** sie nichtionische Vinylpyrrolidonpolymere mit einem relativen Molekulargewicht von vorzugsweise 25.000 bis 30.000 in einer Menge von vorzugsweise 1,3 bis 1,55 Gew.-% enthält.

8. Arzneizusammensetzung mit kontrollierter Freisetzung enthaltend Tramadolhydrochlorid nach Ansprüchen 1, 4 and 6, **dadurch gekennzeichnet, dass** sie Substanzen aus der Gruppe der Salze der höheren Fettsäuren mit Erdalkalimetallen in einer Menge von 1,5 bis 3,2 Gew.-% enthält.

9. Arzneizusammensetzung mit kontrollierter Freisetzung enthaltend Tramadolhydrochlorid nach Anspruch 8, **dadurch gekennzeichnet, dass** sie Substanzen aus der Gruppe der Salze der höheren Fettsäuren mit Erdalkalimetallen, vorzugsweise Magnesiumstearat, in einer Menge von vorzugsweise 1,8 bis 2,8 Gew.-% enthält.

10. Arzneizusammensetzung mit kontrollierter Freisetzung enthaltend Tramadolhydrochlorid nach den Ansprüchen 1, 4, 6 und 8, **dadurch gekennzeichnet, dass** sie Siliciumoxide in einer Menge von 1 bis 3,0 Gew.-% enthält.

11. Arzneizusammensetzung mit kontrollierter Freisetzung enthaltend Tramadolhydrochlorid nach Anspruch 10, **dadurch gekennzeichnet, dass** sie Siliciumoxide, vorzugsweise kolloidales Siliciumdioxid, in einer Menge von 1,1 bis 2,1 Gew.-% enthält.

12. Verfahren zur Herstellung der Arzneizusammensetzung nach den Ansprüchen 1 bis 11, **dadurch gekennzeichnet, dass** der wirkstoff in Mischung mit dem Glycerinester der Docosansäure mit einer Partikelgröße von 1 bis 100 µm in einer Menge von 10 bis 53 Gew.-% und mit Alkalisalzen der Phosphorsäure bewegt wird, wobei die Mischung mit einer Lösung eines nichtionischen Vinylpyrrolidonpolymers in einer Mischung aus Wasser und Ethylalkohol befeuchtet wird und die Mischung agglomeriert wird.

13. Verfahren zur Herstellung der Arzneizusammensetzung nach Anspruch 12, **dadurch gekennzeichnet, dass** der Wirkstoff in Mischung mit dem Glycerinester der Docosansäure mit einer Partikelgröße von 1,5 bis 60 µm in einer Menge von 28 bis 47 Gew.-% zusammen mit Alkalisalzen der Phosphorsäure, vorzugsweise Calciumphosphatdihydrat, in einer Menge von 20 bis 41 Gew.-%, vorzugsweise von 24 bis 39 Gew.-%, bewegt wird.

14. Verfahren zur Herstellung der Arzneizusammensetzung nach den Ansprüchen 12 und 13, **dadurch gekennzeichnet, dass** die Mischung aus dem Wirkstoff, dem Glycerinester der Docosansäure und Alkalisalzen der Phosphorsäure mit einer Lösung aus einem nichtionischen Vinylpyrrolidonpolymer mit einem relativen Molekulargewicht von 9000 bis 90.000, vorzugsweise 25.000 to 30.000, in einer Menge von 1,15 bis 1,75 Gew.-%, vorzugsweise 1,3 bis 1,55 Gew.-%, befeuchtet wird.

15. Verfahren zur Herstellung der Arzneizusammensetzung nach den Ansprüchen 12 bis 14, **dadurch gekennzeichnet, dass** die Mischung aus dem Wirkstoff, dem Glycerinester der Docosansäure und Alkalisalzen der Phosphorsäure mit einer Lösung eines nichtionischen Vinylpyrrolidonpolymers in einer Mischung aus Wasser und Ethylalkohol in einer Menge von 30 bis 70 Gew.-%, vorzugsweise 40 bis 60 Gew.-%, befeuchtet wird.

16. Verfahren zur Herstellung der Arzneizusammensetzung nach den Ansprüchen 12 to 15, **dadurch gekennzeichnet, dass** die Mischung bewegt und agglomeriert wird, wobei das erhaltene Agglomerat in geeigneter Weise durch Fließbetttrocknung, Kammertrocknung oder Vakuumtrocknung getrocknet wird, so dass die Mischung einen Feuchtigkeitsgehalt von 0,2 bis 1,5 %, vorzugsweise von 0,5 bis 1,2 %, aufweist.

17. Verfahren zur Herstellung der Arzneizusammensetzung nach den Ansprüchen 12 to 16, **dadurch gekennzeichnet, dass** das getrocknete Agglomerat auf eine für den Tablettierungsprozess zweckmäßige Partikelgröße eingestellt wird und Substanzen aus der Gruppe der Salze der höheren Fettsäuren mit Erdalkalimetallen und/oder Siliciumoxide beigemischt werden, wobei die Mischung bis zur Homogenität bewegt wird.

18. Verfahren zur Herstellung der Arzneizusammensetzung nach Anspruch 17, **dadurch gekennzeichnet, dass** das getrocknete Agglomerat auf eine für den Tablettierungsprozess zweckmäßige Partikelgröße eingestellt wird und dass Substanzen aus der Gruppe der Salze der höheren Fettsäuren mit Erdalkalimetallen, vorzugsweise Magnesiumstearat, in einer Menge von 1,5 bis 3,2 Gew.-%, vorzugsweise 1,8 bis 2,8 Gew.-%, und/oder Siliciumoxide, vorzugsweise kolloidales Siliciumdioxid, in einer Menge von 1 bis 3 Gew.-%, vorzugsweise von 1,1 bis 2,1 Gew.-%, beigemischt werden.

19. Verfahren zur Herstellung der Arzneizusammensetzung nach den Ansprüchen 12 bis 18, **dadurch gekennzeichnet, dass** die hergestellte Mischung zu Tabletten tablettiert wird, die linsenförmig sind oder eine flache, längliche Form oder eine andere Form aufweisen, so dass die Bruchfestigkeit der Tabletten im Bereich zwischen 40 und 110 N, vorzugsweise von 50 bis 90 N, liegt.

## Revendications

1. Composition pharmaceutique à libération contrôlée contenant du chlorhydrate de tramadol, **caractérisée en ce qu'**elle contient 100 à 200 mg du principe actif dans un mélange composé d'ester de glycéryle micronisé de l'acide docosanoïque d'une taille de particule comprise entre 1 et 100 micromètres, dans une quantité de 10 à 53% en poids, de sels alcalins d'acide phosphorique, de polymères de vinylpyrrolidone non ioniques, et de substances appartenant au groupe composé de sels d'acides gras supérieurs avec des métaux alcalino-terreux et d'oxydes de silicium.

2. Composition pharmaceutique à libération contrôlée contenant du chlorhydrate de tramadol selon la revendication 1, **caractérisée en ce que** 90% de la taille de particule de l'ester de glycéryle de l'acide docosanoïque est comprise entre 1,5 et 60 micromètres.

3. Composition pharmaceutique à libération contrôlée contenant du chlorhydrate de tramadol selon les revendications 1 et 2, **caractérisée en ce que** le contenu de l'ester de glycéryle de l'acide docosanoïque est compris entre 28 et 47% en poids.

4. Composition pharmaceutique à libération contrôlée contenant du chlorhydrate de tramadol selon la revendication 1, **caractérisée en ce qu'**elle contient des sels alcalins de l'acide phosphorique, de préférence du dihydrate de phosphate de calcium dans une quantité comprise entre 20 et 41% en poids.

5. Composition pharmaceutique à libération contrôlée contenant du chlorhydrate de tramadol selon la revendication 4, **caractérisée en ce qu'**elle contient du dihydrate de phosphate de calcium de préférence dans une quantité comprise entre 24 et 39% en poids.

6. Composition pharmaceutique à libération contrôlée contenant du chlorhydrate de tramadol selon les revendications 1 et 4, **caractérisée en ce qu'**elle contient des polymères de vinylpyrrolidone non ioniques dont le poids moléculaire relatif est compris entre 9000 et 90.000, dans une quantité comprise entre 1,15 et 1,75% en poids.

7. Composition pharmaceutique à libération contrôlée contenant du chlorhydrate de tramadol selon la revendication 6, **caractérisée en ce qu'**elle contient des polymères de vinylpyrrolidone non ioniques dont le poids moléculaire relatif est compris de préférence entre 25 000 et 30.000, dans une quantité comprise de préférence entre 1,3 et 1,55% en poids.

8. Composition pharmaceutique à libération contrôlée contenant du chlorhydrate de tramadol selon les revendications 1, 4 et 6, **caractérisée en ce qu'**elle contient des substances appartenant au groupe composé de sels d'acides gras supérieurs avec des métaux alcalino-terreux dans une quantité comprise entre 1,5 et 3,2% en poids.

9. Composition pharmaceutique à libération contrôlée contenant du chlorhydrate de tramadol selon la revendication 8, **caractérisée en ce qu'**elle contient des substances appartenant au groupe de sels d'acides gras supérieurs avec des métaux alcalino-terreux, de préférence le stéarate de magnésium, dans une quantité comprise entre de préférence 1,8 et 2,8% en poids.

10. Composition pharmaceutique à libération contrôlée contenant du chlorhydrate de tramadol selon les revendications 1, 4, 6 et 8, **caractérisée en ce qu'**elle contient des oxydes de silicium dans une quantité comprise entre 1 et 3,0% en poids.

11. Composition pharmaceutique à libération contrôlée contenant du chlorhydrate de tramadol selon la revendication 10, **caractérisée en ce qu'**elle contient des oxydes de silicium, de préférence de la silice colloïdale, dans une quantité comprise entre 1,1 et 2,1 % en poids.

12. Procédé pour la préparation de la composition pharmaceutique selon les revendications 1 à 11, **caractérisé en ce que** le principe actif est agité dans un mélange composé d'ester de glycéryle micronisé de l'acide docosanoïque d'une taille de particule comprise entre 1 et 100 µm, dans une quantité comprise entre 10 et 53% en poids, et avec des sels alcalins de l'acide phosphorique, dans lequel le mélange est humidifié à l'aide d'une solution composée d'un polymère de vinylpyrrolidone non ionique dans un mélange d'eau et d'alcool éthylique, et ledit mélange est aggloméré.

13. Procédé pour la préparation de la composition pharmaceutique selon la revendication 12, **caractérisé en ce que** le principe actif est agité dans un mélange composé d'ester de glycéryle de l'acide docosanoïque d'une taille de particule comprise entre 1,5 et 60 µm, dans une quantité comprise entre 28 et 47 % en poids, et avec des sels alcalins de l'acide phosphorique, de préférence du dihydrate de phosphate de calcium, dans une quantité comprise entre 20 et 41% en poids, de préférence entre 24 et 39 % en poids.

14. Procédé pour la préparation de la composition pharmaceutique selon les revendications 12 et 13, **caractérisé en ce que** le mélange composé du principe actif, dudit ester de glycéryle de l'acide docosanoïque et des sels alcalins de l'acide phosphorique est humidifié à l'aide d'une solution composée d'un polymère de vinylpyrrolidone non ionique d'un poids moléculaire relatif compris entre 9000 et 90.000, de préférence entre 25.000 et 30.000, dans une quantité comprise entre 1,15 et 1,75% en poids, de préférence entre 1,3 et 1,55% en poids.

15. Procédé pour la préparation de la composition pharmaceutique selon les revendications 12 à 14, **caractérisé en ce que** le mélange composé du principe actif, dudit ester de glycéryle de l'acide docosanoïque et des sels alcalins de l'acide phosphorique est humidifié à l'aide d'une solution composée d'un polymère de vinylpyrrolidone non ionique dans un mélange d'eau et d'alcool éthylique dans une quantité comprise entre 30 et 70% en poids, de préférence entre 40 et 60% en poids.

16. Procédé pour la préparation de la composition pharmaceutique selon les revendications 12 à 15, **caractérisé en ce que** le mélange est agité et aggloméré, l'agglomérat obtenu est séché de manière adéquate par séchage en lit fluidisé, par séchage en chambre ou par séchage sous vide, de telle sorte que le mélange contienne entre 0,2 et 1,5, de préférence entre 0,5 et 1,2% d'humidité.

17. Procédé pour la préparation de la composition pharmaceutique selon les revendications 12 à 16, **caractérisé en ce que** l'agglomérat séché est ajusté à une taille de particule convenant au processus de mise en comprimés, et des substances appartenant au groupe composé de sels d'acides gras supérieurs avec des métaux alcalino-terreux et/ou d'oxydes de silicium sont incorporées, et le mélange est agité jusqu'à homogénéité.

18. Procédé pour la préparation de la composition pharmaceutique selon la revendication 17, **caractérisé en ce que** l'agglomérat séché est ajusté à une taille de particule convenant au processus de mise en comprimés, et des substances appartenant au groupe composé de sels d'acides gras supérieurs avec des métaux alcalino-terreux sont incorporées, de préférence le stéarate de magnésium, dans une quantité comprise entre 1,5 et 3,2% en poids, de préférence entre 1,8 et 2,8% en poids, et/ou d'oxydes de silicium, de préférence de la silice colloïdale dans une quantité comprise entre 1 et 3% en poids, de préférence entre 1,1 et 2,1 % en poids.

19. Procédé pour la préparation de la composition pharmaceutique selon les revendications 12 à 18, **caractérisé en ce que** le mélange préparé est produit sous forme de comprimés présentant une forme lenticulaire, oblongue plate ou une autre forme de sorte que les comprimés présentent une résistance à la rupture comprise entre 40 et 110 N, de préférence entre 50 et 90 N.
